# EUROPEAN PATENT APPLICATION

(11) **EP 4 686 482 A1**
(43) Date of publication of application: **04.02.2026**
(21) Application number: 24191507.3
(22) Date of filing: 29.07.2024
(51) Int. Cl.: A61L 2/08, B65B 55/00, B67C 7/00

(54) **PROCESS FOR PREPARING A STERILE PACKAGE OF AN AQUEOUS PERACETIC ACID SOLUTION USING X-RAY RADIATION**

(71) Applicant: Bioxal SA, 71100 Chalon sur Saône (FR)
(72) Inventor: DEPREZ, Alicia, Chalon sur Saône (FR); DELANNOYE, Amandine, Chalon sur Saône (FR); ROUELLE, Ammie, Chalon sur Saône (FR); LALEVÉE, Samuel, Chalon sur Saône (FR)
(74) Representative: LKGLOBAL Partnerschaftsgesellschaft mbB

(57) **Abstract**

The invention relates to a process for preparing a sterile packaging of an aqueous solution of peracetic acid by decontamination of the surface of the packaging, the process comprising the following steps: (i) a first step during which the aqueous solution of peracetic acid is introduced into the packaging to obtain the packaging containing the aqueous solution of peracetic acid; (ii) a second step during which the packaging containing the aqueous solution of peracetic acid is closed at the end of the first step (i); and (iii) a third step during which the closed packaging containing said aqueous solution of peracetic acid, resulting from step b), is exposed to X-ray radiation at an adsorbed radiation dose of between 8,000 Gray and 50,000 Gray.

## Description

### Technical Field

The invention relates to a process for preparing a sterile packaging of an aqueous solution of peracetic acid by decontamination of the surface of the packaging.

### Background Art

Ready-to-use aqueous solutions of peracetic acid are widely known and are marketed for disinfecting medical and surgical equipment, in particular for equipment which cannot be heat sterilized. For instance, such peracetic acid containing solutions have been developed for disinfecting endoscopes, contact lenses and small dental and ophthalmic equipment. They are described, for example, in EP 0 873 687 A1 and FR 2 855 759 A1.

However, for good disinfecting power of a peracetic acid containing solution it is of importance that the solution itself and particularly including its packaging is not contaminated with any potential pathogenic microorganism. One way of obtaining sterile packaging is to subject it to gamma irradiation, for example as described in EP 5 83 695 A1 and FR 2 756 259 A1, or to UV-irradiation as described for instance in US 2004/0234569 A1. Gamma irradiation, however, has the drawback that highly specialized equipment is needed. Such equipment usually comprising radioactive material is very expensive with limited availability. Usually, gamma irradiation must be performed by specialized firms which, however, requires that any packaging to be gamma irradiated needs to be transported to such specialized firm which is tedious and costly. UV-irradiation may have the drawback that due to the lower energetic UV radiation disinfecting is not sufficient if the irradiation time was not chosen long enough. Also UV-radiation may be absorbed even by certain plastic materials compromising the disinfecting efficiency.

Moreover, peracetic acid containing solutions are known to be relatively unstable, in particular when exposed to electromagnetic radiation, in particular to the sun. In addition, peracetic acid containing solutions are also known to be unstable when exposed to gamma radiation, as described in US 2010/0196505 A1. Moreover, it is known that irradiation of hydrogen peroxide leads to the formation of free HO₂⁻ or HO⁻ radicals and, as a consequence, to peroxide decomposition.

Hence, there is a need for improved methods for decontamination of disinfectant solutions. In particular, it would be desirable if such improved method achieves excellent decontamination results, ensures sufficient sterility even if the packaging containing the disinfectant solution is kept for a certain time on the shelf ("*shelf-life*")*,* does not decompose the peracetic acid containing solution, can be quickly applied, and/or does not need highly complex equipment such as gamma ray producing equipment.

The present invention is therefore directed to providing such improved method for decontamination of disinfectant solutions providing at least some and preferably all of the above described advantages.

### Summary of Invention

The present invention is directed to a process for preparing a sterile packaging of an aqueous solution of peracetic acid by decontamination of the surface of the packaging, the process comprising the following steps:
(i) a first step during which the aqueous solution of peracetic acid is introduced into the packaging to obtain the packaging containing the aqueous solution of peracetic acid;
(ii) a second step during which the packaging containing the aqueous solution of peracetic acid is closed at the end of the first step (i);
(iii) a third step during which the closed packaging containing said aqueous solution of peracetic acid, resulting from step b), is exposed to X-ray radiation at an adsorbed radiation dose of between 8,000 Gray and 50,000 Gray.

### Description of Embodiments

The present invention is directed to a process for preparing a sterile packaging of an aqueous solution of peracetic acid by decontamination of the surface of the packaging, the process comprising the following steps:
(i) a first step during which the aqueous solution of peracetic acid is introduced into the packaging to obtain the packaging containing the aqueous solution of peracetic acid;
(ii) a second step during which the packaging containing the aqueous solution of peracetic acid is closed at the end of the first step (i);
(iii) a third step during which the closed packaging containing said aqueous solution of peracetic acid, resulting from step b), is exposed to X-ray radiation at an adsorbed radiation dose of between 8,000 Gray and 50,000 Gray.

In the first step an aqueous solution of peracetic acid is introduced into the packaging to obtain the packaging containing the aqueous solution of peracetic acid.

The aqueous solution of peracetic acid as such is not limited and in principle any peracetic acid containing solution can be used. For instance, the aqueous solution of peracetic acid may comprise peracetic acid, hydrogen peroxide and acetic acid. These three components co-exist in a chemical equilibrium, i.e. peracetic acid on the one side and hydrogen peroxide and acetic acid on the other side. In other words, the presence of acetic acid in such solution helps to maintain the chemical equilibrium (also called herein "the chemical balance") of the aqueous solution of peracetic acid. The aqueous solution of peracetic acid may be a ready-to use composition or a concentrate. Ready to use compositions usually contain up to 6% by weight peracetic acid. Concentrates usually contain more than 6% by weight peracetic acid.

The amount of peracid acid in the composition may be from 0.01% by weight to 20% by weight, such as from 0.1% by weight to 15% by weight, from 0.5% by weight to 12% by weight, or from 1% by weight to 10% by weight. In some embodiments, the amount of peracid acid in the composition may be preferably from 1% by weight to 9% by weight, such as from 2% by weight to 9% by weight, from 3% by weight to 8.5% by weight or from 5% by weight to 8.5% by weight, and more preferably from 5.5% by weight to 8% by weight or from 6% by weight to 8% by weight, the % by weight being based on the total weight of the aqueous solution of peracetic acid. In other embodiments, the amount of peracid acid in the composition may be preferably from 0.01% by weight to 6% by weight, such as from 0.01% by weight to 5% by weight, from 0.05% by weight to 5% by weight, or from 0.1% by weight to 4% by weight, the % by weight being based on the total weight of the aqueous solution of peracetic acid.

The amount of hydrogen peroxide in the composition may be from 1% by weight to 40% by weight, such as from 1% by weight to 35% by weight, or from 1% by weight to 30% by weight. In some embodiments, the amount of hydrogen peroxide in the composition may be from 5% by weight to 30% by weight, or from 7% by weight to 30% by weight, preferably from 10% by weight to 28% by weight, such as from 12% by weight to 28% by weight, or from 15% by weight to 28%by weight, and more preferably from 15 to 25% by weight, the % by weight being based on the total weight of the aqueous solution of peracetic acid. In other embodiments, the amount of hydrogen peroxide in the composition may be from 1% by weight to 12% by weight, preferably from 1% by weight to 10% by weight, such as from 2% by weight to 9% by weight, from 3% by weight to 8% by weight, or from 3% by weight to 5% by weight, the % by weight being based on the total weight of the aqueous solution of peracetic acid.

The amount of acetic acid in the composition is adjusted to an amount which is necessary to maintain the chemical balance of the aqueous solution of peracetic acid. For instance, the amount of acid required to maintain the chemical equilibrium of the aqueous peracetic acid solution may be from 0.1% by weight to 45% by weight.

The aqueous peracetic acid solution used in the first step of the process of the present invention may further comprise one or more additives, such as one or more corrosion-inhibiting agents such as alkaline or alkaline-earth salts of phosphoric acid, for instance sodium hydrogen phosphate or sodium dihydrogen phosphate, one or more free-radical stabilizers and/or sequestrants, such as hydrochloric acid, sulfuric acid, phosphoric acid, pyrophosphate, dipicolinic acid, phosphonic acids or butyl hydroxy toluenes, one or more coloring agents compatible with peracetic acid, one or more fatty amine oxides, such as coodimethylamine oxide, tetradecyl dimethylamine oxide or dimethyl stearylamine oxide, and/or one or more nonionic surfactants, such as a polyethoxylated and/or polypropoxylated fatty alcohol such as the commercial available product Genapol^{™}2908D, which is a mixture of C11, C13 and C15 alcohols (6 to 70E, 30P). The one or more additives can be present in a total amount up to 10% by weight, such as up to 7% by weight, up to 5% by weight, up to 3% by weight, up to 2% by weight, up to 1% by weight, up to 0.8% by weight, or up to 0.5% by weight, the % by weight being based on the total weight of the aqueous solution of peracetic acid. In some embodiments, the one or more additives can be present in a total amount from 0.0001 to 10% by weight, such as from 0.001 to 7% by weight, from 0.001 to 5% by weight, from 0.001 to 3% by weight, from 0.001 to 1% by weight, from 0.001 to 1% by weight, from 0.001 to 0.5% by weight, or from 0.01 to 0.5% by weight.

The aqueous peracetic acid solution may comprise the one or more corrosion-inhibiting agents in an amount of 0.01% by weight to 5% by weight, such as from 0.01% by weight to 1% by weight, or 0.01% by weight to 0.5% by weight, the one or more free-radical stabilizers and/or sequestrants in an amount of from 0.001% by weight to 5% by weight, such as from 0.01% by weight to 1% by weight, or 0.01% by weight to 0.5% by weight, the one or more coloring agents in an amount of from 0.0001% by weight to 0.05% by weight, the one or more fatty amine oxides in an amount of from 0.001% by weight to 1% by weight, such as from 0.001% by weight to 0.5% by weight, or 0.01% by weight to 0.1% by weight, and/or the one or more nonionic surfactants in an amount of from 0.001% by weight to 0.5% by weight, such as from 0.01% by weight to 1% by weight, or 0.01% by weight to 0.5% by weight, the % by weight each being based on the total weight of the aqueous solution of peracetic acid. In some embodiments, the aqueous peracetic acid solution comprises all above-mentioned additives, for instance each in the respective amount ranges as specified above.

The balance of the aqueous solution of peracetic acid is water.

Hence, in some embodiments, the aqueous solution of peracetic acid used in the first step of the process of the present invention comprises
a) from 0.01% by weight to 10% by weight, preferably from 1 % by weight to 9% by weight, and more preferably from 5.5 to 8% by weight of peracetic acid, such as from 6 to 8% by weight of peracetic acid;
b) from 1% by weight to 30% by weight, preferably from 10% by weight to 28% by weight, and more preferably from 15 to 25% by weight of hydrogen peroxide, and
c) acetic acid in an amount necessary to maintain the chemical balance of the aqueous solution of peracetic acid,
wherein the % by weight are based on the total weight of the aqueous solution of peracetic acid.

In some embodiments, the aqueous solution of peracetic acid used in the first step of the process of the present invention comprises
a) from 0.01% by weight to 10% by weight, preferably from 1 % by weight to 9% by weight, and more preferably from 5.5 to 8% by weight of peracetic acid, such as from 6 to 8% by weight of peracetic acid;
b) from 1% by weight to 30% by weight, preferably from 10% by weight to 28% by weight, and more preferably from 15 to 25% by weight of hydrogen peroxide, and
c) acetic acid in an amount necessary to maintain the chemical balance of the aqueous solution of peracetic acid, and
d) in total up to 10% by weight, preferably from 0.001 to 10% by weight, of one or more further additives, and more preferably from 0.001% by weight to 5% by weight of the one or more corrosion-inhibiting agents and/or from 0.001% by weight to 5% by weight of the one or more free-radical stabilizers and/or sequestrants,
wherein the % by weight are based on the total weight of the aqueous solution of peracetic acid.

In further embodiments, the aqueous solution of peracetic acid used in the first step of the process of the present invention comprises
a) from 0.01% by weight to 6% by weight, preferably from 0.01% by weight to 5% by weight of peracetic acid,
b) from 1% by weight to 12% by weight, preferably from 1% by weight to 10% by weight of hydrogen peroxide, and
c) acetic acid in an amount necessary to maintain the chemical balance of the aqueous solution of peracetic acid,
wherein the % by weight are based on the total weight of the aqueous solution of peracetic acid.

In even further embodiments, the aqueous solution of peracetic acid used in the first step of the process of the present invention comprises
a) from 0.01% by weight to 6% by weight, preferably from 0.01% by weight to 5% by weight of peracetic acid,
b) from 1% by weight to 12% by weight, preferably from 1% by weight to 10% by weight of hydrogen peroxide, and
c) acetic acid in an amount necessary to maintain the chemical balance of the aqueous solution of peracetic acid,
d) in total up to 1% by weight preferably from 0.001 to 1% by weight, of one or more further additives, and more preferably from 0.001% by weight to 0.5% by weight of the one or more corrosion-inhibiting agents and/or from 0.001% by weight to 0.5% by weight of the one or more free-radical stabilizers and/or sequestrants,
wherein the % by weight are based on the total weight of the aqueous solution of peracetic acid.

In some embodiments, the balance of any of the above described aqueous solutions of peracetic acid is water.

The packaging as such is in principle not limited and any packing suitable to contain an aqueous solution of peracetic acid may be used. This may be for instance any vessel in the form of a bottle, flask, can, canister, etc. Moreover, the packaging can be made from any suitable material, such as plastics or metal. Preferably, the packaging is a bottle or canister made from plastics, such as polyethylene or polypropylene, for instance with a volume of from 0.01 to 5 liters, such as from 0.03 to 3 liters or from 0.04 to 2 liters, preferably from 0.05 to one liter, such as from 0.1 to on liter, from 0.2 to one liter, or from 0.5 to 1 liter. In some embodiments, the packaging is a sprayer, preferably a manual sprayer, with a volume of from 0.01 to 5 liters, such as from 0.03 to 3 liters or from 0.04 to 2 liters, preferably from 0.05 to one liter, such as from 0.1 to on liter, from 0.2 to one liter, or most preferably from 0.5 to one liter. In some embodiments, the packaging is an "Airless" type manual sprayer with a volume from 0.01 to 5 liters, such as from 0.03 to 3 liters or from 0.04 to 2 liters, preferably from 0.05 to one liter, such as from 0.1 to on liter, from 0.2 to one liter, or most preferably from 0.5 to one liter.

In some embodiments, the closed packaging is a plastic bottle or a plastic canister and multiple plastic bottles or plastic canisters are packed together in a pallet or a box, preferably a carton box.

The process of the present invention further comprises a second step during which the packaging containing the aqueous solution of peracetic acid is closed at the end of the first step (i).

In some embodiments, the packaging containing said aqueous solution of peracetic acid is closed with a device, such as a lid, a cap. etc. In some embodiments, the packaging containing said aqueous solution of peracetic acid is closed with a device comprising a vent. The use of a vent may ensure that no excessive built-up of pressure in the packaging occurs.

In some embodiments, after step (ii), the packaging containing the aqueous solution is packed in at least two separate bags, preferably plastic bags. Such additional packaging helps to ensure that the packaging containing the aqueous solution of peracetic acid maintains sufficient sterility even if the packaging containing the disinfectant solution is kept for a certain time on the shelf, i.e. is just stored. In further embodiments, each of the at least two separate bags may comprise a vent.

In a third step of the process of the present invention the closed packaging containing the aqueous solution of peracetic acid, resulting from step b), is exposed to X-ray radiation at an adsorbed radiation dose of between 8,000 Gray and 50,000 Gray.

Preferably, the absorbed radiation dose is between 8,000 to 45,000 Gray, such as between 8,000 and 40,000 Gray or 10,000 Gray and 35,000 Gray, more preferably between 15,000 Gray and 30,000 Gray, and even more preferably between 18,000 Gray and 28,000 Gray, such as between 18,000 Gray and 27,000 Gray.

The use of X-ray radiation to achieve a radiation dose as specified above ensures that the radiation as such is sufficiently high-energetic to efficiently sterilize the packaging by killing any potential microorganism. At the same time, by using X-ray radiation there is no need for highly specialized equipment such as equipment for the production of gamma-rays. In addition, X ray irradiators are not dependent on an isotopic source to produce radiation such as gamma ray irradiators. Even further, X-ray radiation is high-energetic enough to not be absorbed by any standard material used for preparing the packaging of an aqueous solution of peracetic acid, in particular not by commonly used plastics.

### Examples

Tests have been performed to irradiate pallets of 60 ml and 500 ml plastic bottles containing aqueous solutions of peracetic acid. Irradiation was performed using X-rays at a total dose of 19.9 ± 0.2 kGray, 20.4 ± 0.2 kGray 25.0 ± 0.2 kGray, 26.9 ± 0.2 kGray, and 27.1± 0.2 kGray. It could be confirmed that the aqueous solutions of peracetic acid were not degraded by the X-ray irradiation. Moreover, it could be confirmed that the sterilization of the bottles was sufficient.

Even further, no endotoxins on the outside of the bottle, inside the bottle and inside the caps could be detected. Endotoxins are components of the wall of certain gram-negative bacteria and complex molecules. These molecules released during the multiplication or destruction of bacteria, persist for a long time in the environment and are resistant to many chemical or physical agents. Endotoxins are found in the same places as gram-negative bacteria and persist in the environment even if these bacteria are destroyed. Bacteria are present wherever moisture and organic or inorganic matter are present. Endotoxins can be emitted into the air during work tasks that produce aerosols. Endotoxins have pathogenic effects indeed they can cause fever and in the worst case (for immunocompromised patients for example) death.

Even further, storage experiments have been performed, wherein microbiological contamination of X-ray irradiated samples after storage under different conditions has been evaluated. 500 ml bottles packed in two separate plastic bags were stored for 24 months at 5°C, 20°C and 30°C and 40 ml bottles packed in two separate plastic bags were stored for 15 months at 5°C, 20°C and 30°C.The 40 ml bottles stored at 30°C were further stored up to 18 months, Storage conditions (time and temperature) are representative for normal use periods and life-time of commercial peracetic acid-containing compositions, i.e. a storage time of 15-24 months is representative for an average standard shelf life of a plastic bottle comprising an aqueous solution of peracetic acid and the temperatures are representative for either fridge storage or storage at ambient temperatures "on the shelf". It could be confirmed that in all storage experiments, after storage the bottles still were sterile inside and did not show any microbiological contamination.

## Claims

1. A process for preparing a sterile packaging of an aqueous solution of peracetic acid by decontamination of the surface of the packaging, the process comprising the following steps:
(i) a first step during which the aqueous solution of peracetic acid is introduced into the packaging to obtain the packaging containing the aqueous solution of peracetic acid;
(ii) a second step during which the packaging containing the aqueous solution of peracetic acid is closed at the end of the first step (i);
(iii) a third step during which the closed packaging containing said aqueous solution of peracetic acid, resulting from step b), is exposed to X-ray radiation at an adsorbed radiation dose of between 8,000 Gray and 50,000 Gray.

2. The process of claim 1, wherein the aqueous solution of peracetic acid comprises
a) from 0.01% by weight to 10% by weight, preferably from 1% by weight to 9% by weight, and more preferably from 5.5 to 8% by weight of peracetic acid,
b) from 1% by weight to 30% by weight, preferably from 10% by weight to 28% by weight, and more preferably from 15 to 25% by weight of hydrogen peroxide,
c) acetic acid in an amount necessary to maintain the chemical balance of the aqueous solution of peracetic acid,
wherein the % by weight are based on the total weight of the aqueous solution of peracetic acid.

3. The process of claim 1, wherein the aqueous solution of peracetic acid comprises
a) from 0.01% by weight to 6% by weight, preferably from 0.01% by weight to 5% by weight of peracetic acid,
b) from 1% by weight to 12% by weight, preferably from 1% by weight to 10% by weight of hydrogen peroxide,
c) acetic acid in an amount necessary to maintain the chemical balance of the aqueous solution of peracetic acid,
wherein the % by weight are based on the total weight of the aqueous solution of peracetic acid.

4. The process of any of claims 1-3, wherein the packaging containing said aqueous solution of peracetic acid is closed with a device comprising a vent.

5. The process of any one of claims 1-4, wherein the radiation dose is 8,000 to 45,000 Gray, such as between 8,000 and 40,000 Gray or between 10,000 Gray and 35,000 Gray, preferably between 15,000 Gray and 30,000 Gray, and more preferably between 18,000 Gray and 28,000 Gray, such as between 18,000 Gray and 27,000 Gray.

6. The process of any one of claims 1-5, wherein the X-rays have a wavelength of from 0.01 to 10 nm.

7. The process of any one of claims 1-6, wherein the closed packaging is a bottle or a canister, preferably a plastic bottle or plastic canister.

8. The process of any one of claims 1-7, wherein the closed packaging is a plastic bottle or a plastic canister and multiple plastic bottles or plastic canisters are packed together in a box, preferably a carton box, which is then exposed to the X-ray radiation.

9. The process of any one of claims 1-8, wherein after step (ii), the packaging containing the aqueous solution is packed in at least two separate bags, preferably plastic bags.

10. The process of claim 9, wherein each of the at least two separate bags comprises a vent.
